# EUROPEAN PATENT APPLICATION

(11) **EP 3 892 368 A1**
(43) Date of publication of application: **13.10.2021**
(21) Application number: 19892655.2
(22) Date of filing: 04.12.2019
(51) Int. Cl.: B01J 20/28, A61L 9/014, B01D 53/38, B01D 53/50, B01D 53/56, B01D 53/70, B01D 53/82, B01J 20/12, B01J 20/18, B01J 20/20, B01J 20/30, C02F 1/28, C02F 1/40

(54) **SINTERED BODY FOR ADSORPTION, PRODUCTION METHOD THEREFOR, AND ADSORPTION DEVICE**

(30) Priority: 05.12.2018 JP 2018227831
(71) Applicant: AJINOMOTO CO., INC., Chuo-ku Tokyo 104-8315 (JP); Nippon Filcon Co., Ltd, Inagi-shi, Tokyo 206-0801 (JP)
(72) Inventor: SEINO, Makoto, Yokohama-shi, Kanagawa 230-0034 (JP); YANO, Hideyuki, Yokohama-shi, Kanagawa 230-0034 (JP); KATOH, Toshifumi, Inagi-shi, Tokyo 206-0801 (JP)
(74) Representative: Strehl Schübel-Hopf & Partner
(86) International application number: PCT/JP2019/047346
(87) International publication number: WO 2020/116491

(57) **Abstract**

[Problem] To adsorb substance to be treated in fluid (7) with higher adsorption capacity and lower pressure loss.

[Solution] An adsorptive sintered compact (20) according to the present invention comprises powder adsorbent materials (1a, 1b), and resin structures (2) in which voids (3) are formed in a three-dimensional network. The powder adsorbent materials (1a, 1b) include free adsorbent materials (1a) free-movably contained in the voids (3) between the resin structures (2), and fixed adsorbent materials (1b) fixed to a surface (2a) of the resin structure (2) and/or at least partly embedded inside the resin structure (2), and the powder adsorbent materials (1a, 1b) are at least one selected from powdered activated carbon, powdered activated clay and zeolite.

## Description

### TECHNICAL FIELD

The present invention relates to a sintered object for adsorption, particularly, to an adsorptive sintered compact which contains powder adsorbent materials for adsorbing substance(s) to be treated in fluid, a production method therefor and an adsorption apparatus.

### BACKGROUND ART

Adsorbent materials such as activated carbon, activated clay and zeolite, etc., are used for various adsorption uses for industrial, household, medical purposes including air purification, dioxin removal, flue gas desulfurization and denitrification, odor removal, waste gas and liquid treatment of factory, advanced water purification, purification of chemicals, decolorization of foods and beverages, water purifiers for home use, air purifiers, refrigerator deodorants, gas masks, etc.

For example, Patent Document 1 discloses an activated carbon cartridge for gas purification in which granular activated carbon having particle diameter of 2.4 to 4.7 mm (2,400 to 4,700 µm) is filled between an inner cylinder and an outer cylinder. In general, "granular" activated carbon refers to activated carbon having larger particle diameter, and fine powder activated carbon having smaller particle diameter is called "powdered" activated carbon. In JIS K 1474, particle diameter indication of 150 µm or more is defined as "granular" activated carbon and less than 150 µm is "powdered" activated carbon. Granular adsorbent material having large particle diameter has small specific surface area (outer surface area per unit mass) as compared with that of fine powder adsorbent material, so that in order to obtain the same level of adsorption performance as when powder adsorbent material is used, it is necessary to increase the amount of granular adsorbent material, whereby when granular adsorbent material is used as filter by filling in cartridge of adsorption apparatus, cartridge or adsorption apparatus becomes large. Also, when granular adsorbent material is tried to be filled tightly in vessel in order to heighten adsorption performance, the filling volume is likely to vary from vessel to vessel as compared with that of powder adsorbent material, so that there is a problem that it is not easy to maintain constant quality in the manufacture of product such as cartridge for filter. In addition, it sometimes causes inconvenience that pressure loss increases since granular adsorbent material in vessel is pulverized, destructed or the like, due to external forces such as vibration and pressure at the time of transportation or use.

On the other hand, powder adsorbent material such as powdered activated carbon, has larger specific surface area and higher adsorption performance as compared with that of granular adsorbent material, but even if it is used as filter by filling it in vessel such as cartridge, voids between powder adsorbent materials are extremely reduced and fluid is difficult to pass through, and even if it passes through, it causes high pressure loss (high differential pressure) whereby it is not suitable for practical use. For this reason, powder adsorbent material is not suitable for filter by filling in vessel such as cartridge, and therefore, has been conventionally used exclusively for a batch type adsorption process.

As attempt to improve pressure loss in vessel filled with adsorbent material, for example, Patent Document 2 discloses an adsorptive molded product based on agglomerate formed by binding and/or adhering adsorbent material through binder of thermoplastic resin, and discloses a filter to which it is applied. However, in such agglomerate, most of surface of adsorbent material is covered by thermoplastic resin, so that adsorption performance is lowered. Particularly, in powder adsorbent material having small particle diameter, most of its surface tends to be covered by thermoplastic resin, and it is difficult to make agglomerate into desired size due to decrease in pressure loss, so that in Examples of Patent Document 2, granular activated carbon having large particle diameter is used practically.

Further, as attempt to resolve the problem that surface of adsorbent material contained in adsorptive molded product is covered by thermoplastic resin, for example, Patent Document 3 discloses a molded product in which foaming agent is impregnated or adsorbed to highly functional particles such as activated carbon particles and mixed with matrix resin, and mixture is solidified by foaming in liquid or molten state of matrix resin whereby highly functional particles are allowed to exist inside pores generated by foaming.

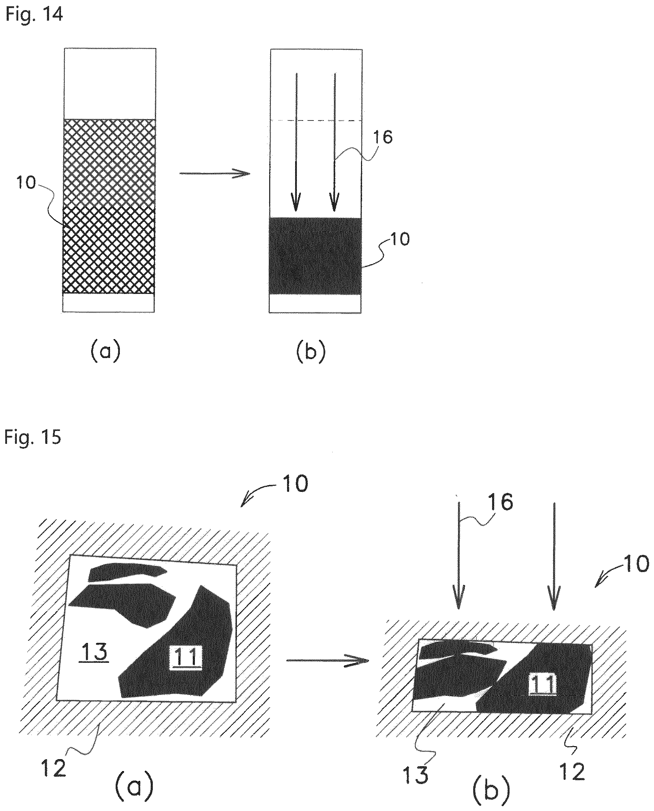

However, when such so-called foamed plastic is used as matrix of adsorbent material, molded product is generally flexible and poor in mechanical strength, particularly, is easily deformed under high pressure and high flow rate, so that it is not suitable for a continuous system adsorption apparatus which adsorbs substance to be treated in fluid. Specifically, for example, as shown in Fig. 14(a) and Fig. 15(a), as compared with molded product 10 before use, molded product 10 at the time of use under high pressure or high flow rate is easily crushed in its entire volume by compressive force 16 in Fig. 14(b) since it is constituted by foamed plastic 12, and as shown in Fig. 15(b), pores 13 tend to shrink as compared with those before use so that density of activated carbon particles 11 becomes high whereby pressure loss increases and flow rate markedly lowers.

In addition, since pores are formed in matrix resin by impregnating foaming agent into adsorbent material, if foaming agent is not sufficiently impregnated into adsorbent material, it is not easy to form suitable pores therein. Further, when content of adsorbent material is increased for the purpose of heightening adsorption performance, pores also increase and mechanical strength tends to lower, so that it is difficult to improve the adsorption efficiency.

Also, an open cell structure which is said to be preferable in obtainable molded product becomes so-called sponge-like structure, and further mechanical strength is lowered, so that there is a problem that it is not suitable for the use of continuous treatment for adsorbing substances to be treated in fluid (7) as used by filling it in vessel such as cartridge as mentioned above.

### PRIOR ART DOCUMENTS

### PATENT DOCUMENTS

Patent Document 1: JP2014-104448A
Patent Document 2: JP2012-508645A
Patent Document 3: JPH01-301732A

### SUMMARY OF THE INVENTION

### PROBLEMS TO BE SOLVED BY THE INVENTION

Thus, an object of the present invention is to provide an adsorptive material which is excellent in adsorption capacity and capable of achieving lower pressure loss simultaneously, and a production method therefor and an adsorption apparatus.

### MEANS TO SOLVE THE PROBLEMS

The present inventors have earnestly studied to accomplish the above-mentioned object, and as a result, they have found that, a specific adsorptive sintered compact comprising: resin structure(s) in which voids are formed to a three-dimensional network; and powder adsorbent materials contained in voids free-movably and simultaneously fixed to a surface of resin structure(s) and/or at least a part thereof embedded inside resin structure(s); is excellent in adsorption capacity and capable of achieving lower pressure loss simultaneously, whereby they have completed the present invention.

That is, the present invention includes the following contents.
[1] An adsorptive sintered compact which comprises:
   powder adsorbent materials (1a, 1b); and
   a resin structure (2) in which voids (3) are formed in a three-dimensional network;
   wherein the powder adsorbent materials (1a, 1b) include:
      a free adsorbent material (1a) free-movably contained in the voids (3) between the resin structures (2); and
      a fixed adsorbent material (1b) fixed to a surface (2a) of the resin structure (2) and/or at least partly embedded inside the resin structure (2); and
   the powder adsorbent materials (1a, 1b) are at least one selected from powdered activated carbon, powdered activated clay and zeolite.
[2] The adsorptive sintered compact described in [1], wherein a mean diameter of the powder adsorbent materials (1a, 1b) is less than 150 µm.
[3] The adsorptive sintered compact described in [1] or [2], wherein the powder adsorbent materials (1a, 1b) are contained in an amount of 25 to 65 weight%.
[4] The adsorptive sintered compact described in any of [1] to [3], wherein a resin raw material of the resin structure (2) is at least one thermoplastic resin selected from polypropylene (PP), polyethylene (PE), polyvinylidene fluoride (PVDF) and ethylene-vinyl acetate (EVA) copolymer.
[5] The adsorptive sintered compact described in any of [1] to [4], wherein a particle diameter of the thermoplastic resin is 10 to 200 µm.
[6] The adsorptive sintered compact described in any of [1] to [5], wherein a plurality of free adsorbent materials (1a) are free-movably contained in at least a part of the voids (3), and the plurality of the adjacent free adsorbent materials (1a) form a flow path (3a) of a fluid (7) between free adsorbent materials (1a) in at least a part of voids (3) without a fixation of the adjacent free adsorbent materials to each other.
[7] The adsorptive sintered compact described in any of [1] to [6], wherein the powder adsorbent material (1a, 1b) is powdered activated carbon having a sharp portion.
[8] The adsorptive sintered compact described in any of [1] to [7], which is used for adsorbing a substance to be treated in the fluid (7).
[9] An adsorption apparatus which comprises a single or a plurality of layers (20a, 20b) of the adsorptive sintered compact (20) described in any of [1] to [8] being loaded in a vessel.
[10] A method for producing an adsorptive sintered compact which comprises steps of:
   mixing a powder adsorbent material which is at least one selected from powdered activated carbon, powdered activated clay and zeolite, with a thermoplastic resin to form an adsorbent material mixture;
   heating the adsorbent material mixture at a temperature higher than a softening point of the thermoplastic resin and lower than a melting point of a raw material of the powder adsorbent material; and
   forming a resin structure (2) in which a plurality of the thermoplastic resins are fused and solidified by cooling to form voids (3) in a three-dimensional network and a free adsorbent material (1a) is free-movably contained in the void (3).
[11] The production method described in [10], wherein a mean diameter of the powder adsorbent material is less than 150 µm.
[12] The production method described in [10] or [11], wherein a content of the powder adsorbent material is 25 to 65 weight% in the adsorbent material mixture.
[13] The production method described in any of [10] to [12], wherein the thermoplastic resin is at least one selected from polypropylene (PP), polyethylene (PE), polyvinylidene fluoride (PVDF) and ethylene-vinyl acetate (EVA) copolymer.
[14] The production method described in any of [10] to [13], wherein a particle diameter of the thermoplastic resin is 10 to 200 µm.

### EFFECTS OF THE INVENTION

According to the present invention, it is possible to provide an adsorptive material which is excellent in adsorption capacity and capable of achieving lower pressure loss simultaneously, and a production method therefor and an adsorption apparatus.

### BRIEF DESCRIPTION OF THE DRAWINGS

Fig. 1 is a partial cross-sectional view showing adsorptive sintered compact of the present invention.
Fig. 2a is an enlarged surface image showing powdered activated carbon as raw material.
Fig. 2b is an enlarged surface image showing powdered activated carbon as raw material.
Fig. 2c is an enlarged surface image showing powdered activated carbon as raw material.
Fig. 2d is an enlarged surface image showing powdered activated clay as raw material.
Fig. 2e is an enlarged surface image showing zeolite as raw material.
Fig. 3 is an enlarged cross-sectional image showing resin structure alone of adsorptive sintered compact.
Fig. 4a is a surface image showing adsorptive sintered compact of the present invention produced from powdered activated carbon.
Fig. 4b is an enlarged surface image showing adsorptive sintered compact of the present invention produced from powdered activated carbon.
Fig. 4c is an enlarged surface image showing adsorptive sintered compact of the present invention produced from powdered activated carbon.
Fig. 4d is an enlarged surface image showing adsorptive sintered compact of the present invention produced from powdered activated clay.
Fig. 4e is an enlarged surface image showing adsorptive sintered compact of the present invention produced from zeolite.
Fig. 5 is a cross-sectional view showing adsorption apparatus loaded with adsorptive sintered compact of the present invention.
Fig. 6 is a partial cross-sectional view showing embodiment in which liquid or gas is passed through adsorptive sintered compact of the present invention.
Fig. 7 is a schematic view showing pressure loss test apparatus.
Fig. 8 is a graph showing results of pressure loss test.
Fig. 9 is a graph showing results of pressure loss test.
Fig. 10 is a schematic view showing liquid flow adsorption test apparatus.
Fig. 11a is a graph showing results of liquid flow adsorption test using coconut shell activated carbon.
Fig. 11b is a graph showing results of liquid flow adsorption test using sawdust activated carbon.
Fig. 12 is a schematic view showing gas adsorption test apparatus.
Fig. 13 is a graph showing results of gas adsorption test.
Fig. 14 is a cross-sectional view showing state in which conventional molded product is loaded in vessel.
Fig. 15 is a partial cross-sectional view showing conventional molded product.

### EMBODIMENTS TO CARRY OUT THE INVENTION

The adsorptive sintered compact of the present invention is provided with powder adsorbent materials (1a, 1b), and resin structure(s) (2) in which voids (3) are formed as a three-dimensional network shape. The resin structure (2) in the present invention can be formed by heating particles of a thermoplastic resin such as powder, granules, pellets, and melting contacted portions of a plurality of thermoplastic resins to form joint portions, whereby the thermoplastic resins are thermally bonded to each other. The resin structures thus obtained have structures in which concave voids sandwiched between convex portions derived from the shape of the thermoplastic resin are formed in three-dimensional network. The powder adsorbent materials (1a, 1b) contain a free adsorbent material (1a) free-movably contained in voids (3) between the resin structures (2), and a fixed adsorbent material (1b) fixed to the surface (2a) of the resin structure (2) and/or at least partly embedded inside the resin structure (2). The powder adsorbent materials (1a, 1b) are constituted by at least one selected from powdered activated carbon, powdered activated clay and zeolite.

The adsorptive sintered compact of the present invention is useful particularly for adsorbing substances to be treated in a fluid (7). When the fluid (7) is passed through the adsorptive sintered compact (20), the entire surface of the free adsorbent material (1a) which is not fixed to the resin structure (2) and not joined, comes into direct contact with the fluid (7), so that it can generate an action of capturing and adsorbing the substances to be treated in the fluid (7). In addition, in the present invention, not only the free adsorbent material (1a) of the voids (3), but also the fixed adsorbent material (1b) is provided in the resin structure (2), so that the adsorptive sintered compact (20) of the present invention can ensure larger adsorption area as compared with the conventionally existed almost all adsorbent materials including the type in which the entire surface or a part of surface is coated by matrix resin or the type in which the adsorbent materials exist only in voids, whereby the adsorption efficiency is markedly increased, and improvement in the adsorption performance can be accomplished.

Further, when the fluid (7) is passed through the adsorptive sintered compact (20), due to voids (3) being formed in the three-dimensional network shape, the free adsorbent materials (1a) are freely movable while the movement thereof is restricted with a certain extent and the agglomeration thereof is suppressed. Therefore, it is possible to suppress clogging of voids (3) and gap (4) between the resin structures (2), and as a result, even when the powder adsorbent materials (1a, 1b) are used, the increase in pressure loss in adsorptive sintered compact (20) is suppressed, and an action continuously adsorbing substances to be adsorbed in the fluid (7) can occur for a long period of time.

Moreover, the substances to be adsorbed captured by the free adsorbent material (1a) can be removed from the flowing and moving free adsorbent materials (1a) if the conditions are selected, and the adsorptive sintered compact (20) can be reused. That is, for removing the adsorbed substances, for example, a method can be selected that the adsorptive sintered compact (20) is heated within a structurally maintainable range, and extracted by adding a good solvent of the adsorbed substance. In addition, it is also possible to select a method in which removal is facilitated by reducing a pressure when it is a gas phase or by changing liquid properties such as pH and a concentration of salt when it is a liquid phase. Furthermore, a plurality of these methods capable of using in parallel can be used in combination. In the adsorptive sintered compact of the present invention, in order to enhance removal ability of the substance to be adsorbed, it is possible to carry metals such as silver, copper, nickel, metal oxides thereof, non-volatile chemicals such as acids, bases, on the raw material of the powder adsorbent material in advance. Further, these can be directly carried on the adsorptive sintered compact.

In the adsorption apparatus of the present invention, a single or a plurality of layers (20a, 20b) of the adsorptive sintered compact (20) is loaded in a vessel. As compared with the case where the powdered activated carbon is directly filled in the vessel, the adsorption apparatus (30) is excellent in strength characteristics due to the rigid resin structure (2), and a certain form and voids (3) can be maintained at the time of transportation and use. Further, even if a plurality of layers (20a, 20b) of the adsorptive sintered compact (20) are loaded, the respective layers (20a, 20b) do not mix.

A method for producing the adsorptive sintered compact of the present invention comprises steps of: mixing at least one powder adsorbent raw material selected from powdered activated carbon, powdered activated clay and zeolite, with a thermoplastic resin to form an adsorbent material mixture; heating the adsorbent material mixture at a temperature higher than the softening point of the thermoplastic resin and lower than the melting point of the powder adsorbent raw material; and fusing a plurality of the thermoplastic resins and solidifying by cooling to form a resin structure (2).

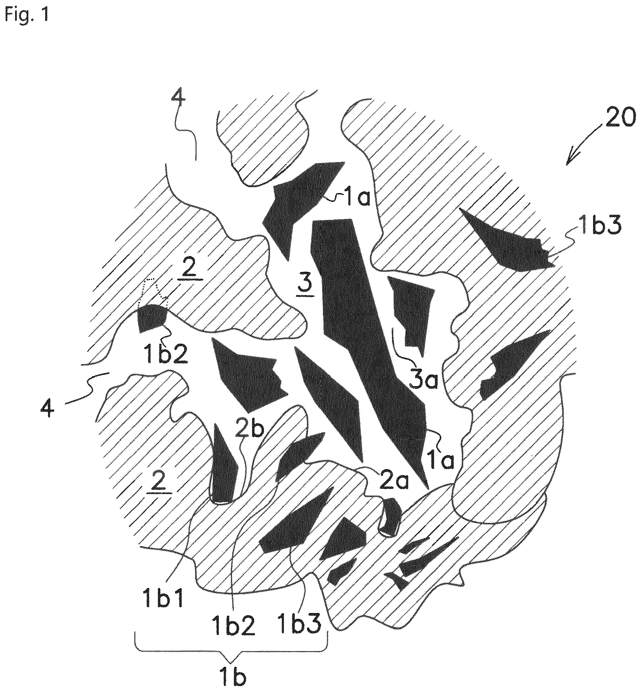

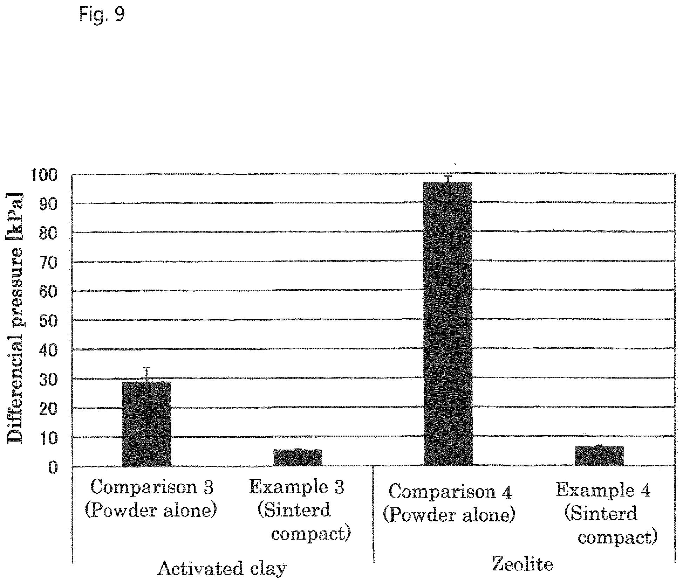

Embodiments of an adsorptive sintered compact and a production method therefor according to the present invention will be explained in more detail below by referring to Fig. 1 to Fig. 13.

Embodiment of an adsorptive sintered compact (20) of the present invention is shown in Fig. 1. Adsorptive sintered compact (20) is provided with powder adsorbent materials (powder adsorbent agents) (1a, 1b) that adsorb substance(s) to be treated in a fluid (7), and resin structure(s) (2) in which voids (cavities) (3) are formed in a three-dimensional network. The substance to be treated is contained in gas or liquid, and may include all components and substances adsorbable to powder adsorbent materials (1a, 1b), for example, color component, odor component, harmful substance, pollutant, heavy metal, valuable metal, toxic component, radioactive component, water, oil, etc.

Powder adsorbent materials (1a, 1b) in the present invention are at least one selected from powdered activated carbon, powdered activated clay, and zeolite. Adsorptive sintered compact (20) may contain other adsorbent substance, for example, acid clay, alumina, silica, silica gel, silica-alumina, vermiculite, perlite, kaolin, diatomaceous earth, sepiolite, etc., to the extent that effects of the present invention can be achieved. Also, in order to further decrease pressure loss when adsorptive sintered compact is used for an adsorption apparatus, Adsorptive sintered compact (20) may contain granular adsorbent materials (adsorbent materials having mean diameter of 150 µm or more) such as granular activated carbon, within a range in which adsorption capacity is not extremely reduced. Activated carbon used for powder adsorbent materials (1a, 1b) is formed by activating a raw material selected from, for example, coconut shell, walnut shell, apricot shell, fruit shell, paddy shell, soybean, coffee, nuts, pistachio, charcoal, sawdust, sawdust, bark, carbon from sawdust, wood material, peat, grass peat, lignite, brown coal, bituminous coal, anthracite, tar, pitch, coke, coal, petroleum, waste tires, waste plastic, synthetic resin, fiber, construction waste and sewage sludge, and the adsorption performance is imparted by innumerable internal micropores. As activation method, for example, a gas activation with water vapor, carbon dioxide, air, etc., and a chemical activation with zinc chloride, phosphoric acid, sulfuric acid, calcium chloride, potassium dichromate, potassium permanganate, sodium hydroxide, etc., can be applied.

A mean diameter of powder adsorbent material in the present invention is not particularly limited as long as the effect of the present invention can be achieved, but it is preferably less than 150 µm. That is, in the present specification, adsorbent material preferably having mean diameter of less than 150 µm is defined to be powder adsorbent material. Also, "powder" adsorbent material having particle diameter indication of less than 150 µm according to JIS K 1474 is also included in powder adsorbent material of the present invention. "Mean diameter" of the present specification is measured by the laser diffraction scattering method based on the Mie scattering theory. Specifically, particle size distribution of powder adsorbent raw material is prepared based on volume basis by the laser diffraction scattering particle analyzer (LA-500 manufactured by HORIBA, Ltd., LMS-2000e manufactured by SEISHIN ENTERPRISE Co., Ltd.), and the median diameter is taken as mean diameter. Incidentally, for example, mean diameter of adsorbent material of less than 150 µm includes any adsorbent materials having median diameter of less than 150 µm. That is, the value of mean diameter merely represents the intermediate value of distribution range, and for example, mean diameter of adsorbent material less than 150 µm does not mean that adsorbent material having diameter of 150 µm or more is not included at all. Mean diameter of powder adsorbent material is more preferably 1 µm or more and less than 150 µm, further preferably 5 µm or more and less than 150 µm, and particularly preferably 15 µm or more and 100 µm or less. Mean diameter of powder adsorbent materials (1a, 1b) contained in adsorptive sintered compact (20) can be adjusted by setting the mean diameter of powder adsorbent material to be used at the time of producing the adsorptive sintered compact (20) within the above numerical range. That is, mean diameter of powder adsorbent material to be used at the production can become mean diameter of powder adsorbent materials (1a, 1b) contained in adsorptive sintered compact (20).

If mean diameter of powder adsorbent materials (1a, 1b) is too small, large amount of fine powder adsorbent materials (1a, 1b) is mixed with resin raw materials of resin structure (2) at the production, so that there is a possibility of lowering the strength of resin structure (2). Also, there is a possibility that fine free adsorbent material (1a) may easily drop out from adsorptive sintered compact (20) with fluid (7) through gap (4) between resin structures (2). Further, there is a possibility that pressure loss is easily increased. If mean diameter is too large (for example, if it is 150 µm or more), the adsorption area of adsorbent material is small and the adsorption performance tends to be lowered. Also, sufficient amount of free adsorbent material (1a) cannot be contained in voids (3), and therefore, the constitution of adsorptive sintered compact (20) tends to be difficult.

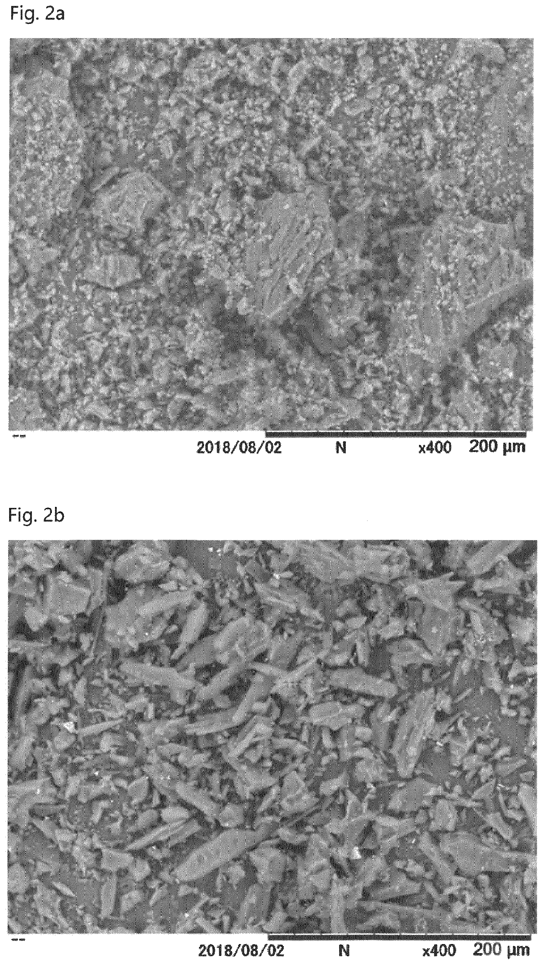

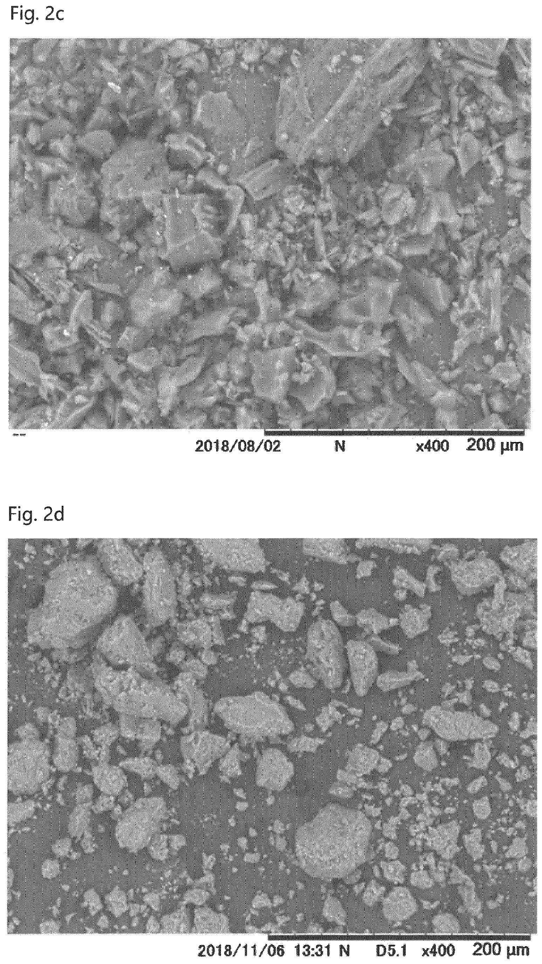

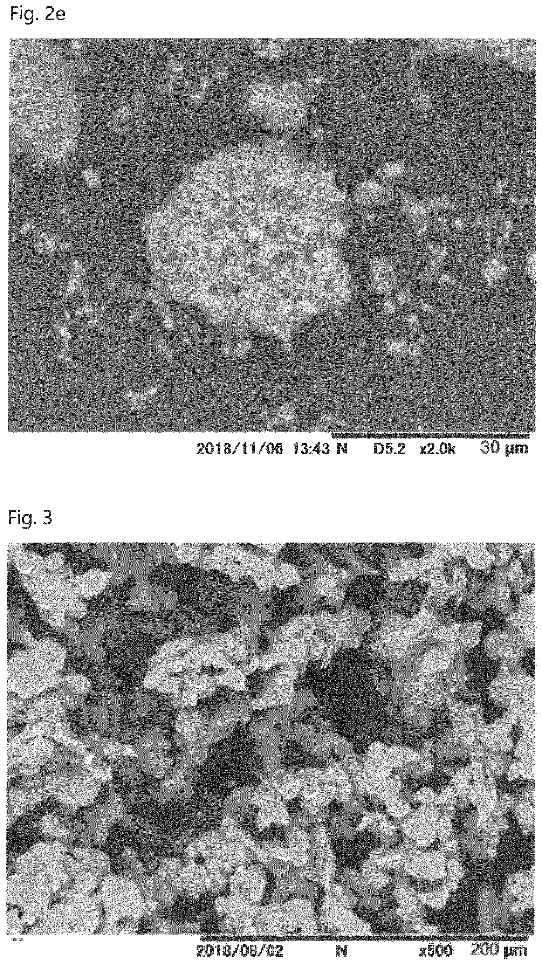

Fig. 2a to Fig. 2e are electron micrograph images showing raw materials of powder adsorbent materials (1a, 1b) for constituting the adsorptive sintered compact (20). In particular, Figs. 2a, 2b and 2c show enlarged surface images (400-fold) of powdered activated carbons obtained by respectively activating: carbide of coconut shell with water vapor; sawdust with chemicals (phosphoric acid); and sawdust with chemicals (zinc chloride). Powdered activated carbon is generally a lumpy, rod-shaped or elongated plate-shaped raw material having sharp portion(s), so that tip of powder adsorbent materials (1a, 1b) locks in surface (2a) and gap (4) of resin structures (2) at the time of freely moving thereof to prevent from flowing out of adsorptive sintered compact (20). Therefore, powder adsorbent materials (1a, 1b) hardly move from void to void (3). Fig. 2d and Fig.2e are enlarged surface images each showing powdered activated clay (400-fold) and zeolite (2,000-fold) as powder adsorbent raw material. Adsorptive sintered compact (20) contains powder adsorbent materials (1a, 1b) of preferably 25 to 65 weight%, and further preferably 30 to 60 weight%. If content of powder adsorbent materials (1a, 1b) is too low, adsorption performance of adsorptive sintered compact (20) tends to be lowered. On the other hand, if content of powder adsorbent materials (1a, 1b) is too high, it has high adsorption capacity, but ratio of resin is small and the strength of entire adsorptive sintered compact (20) tends to be lowered. In the present invention, the content of powder adsorbent materials (1a, 1b) contained in adsorptive sintered compact (20) can be defined as that of powder adsorbent material contained in adsorbent material mixture before sintering to be used in the production thereof, and therefore, can be adjusted by appropriately setting the content of powder adsorbent material in adsorbent material mixture.

Thermoplastic resin is used as resin raw material of resin structure (2). It is preferably at least one thermoplastic resin selected from polypropylene (PP), polyethylene (PE), polyvinylidene fluoride (PVDF) and ethylene-vinyl acetate (EVA) copolymer. Thermoplastic resin as resin raw material of resin structure (2) is used in a particulate solid, and may be in any form such as powder, granules, pellets. Particle diameter of thermoplastic resin particle is preferably 10 to 200 µm. By setting particle diameter of thermoplastic resin within the above-mentioned range, it becomes easy to form size of voids (3) into substantially uniform dimensions without variation. Plasticizer such as adipic acid ester, stabilizer such as epoxy-based compound, antioxidant such as phenol-based compound, can be added to thermoplastic resin depending on the kind and characteristics thereof.

Particle diameter of thermoplastic resin in the present specification is measured by the image analysis method photographed by CCD camera. Specifically, 1,400 to 15,000 randomly dispersed particles of thermoplastic resin are photographed by an image analysis type particle distribution measuring device (VD-3000 manufactured by JASCO International Co., Ltd., PITA-04 manufactured by SEISHIN ENTERPRISE Co., Ltd., etc.) to obtain images which calculate the individual particle diameter D of each particle, and after preparing distribution of D, the median diameter is made particle diameter of thermoplastic resin. Incidentally, individual particle diameter D can be obtained from a maximum diameter D1 of maximum width and a minimum diameter D2 of minimum width of particle in the image and from the arithmetic mean (D1 + D2)/2.

Fig. 3 is a cross-sectional enlarged image (500-fold) by electron microscope of adsorptive sintered compact containing no powder adsorbent materials (1a, 1b), and shows the state of cross-section in which resin raw material alone is sintered to form resin structure and cut it by cutter. White portion of Fig. 3 shows resin structures (2) of continuous tufted and three-dimensional network structure without any corner, and black part inside thereof shows voids (3) in which free adsorbent materials (1a) are to be arranged free-movably.

Powder adsorbent materials (1a, 1b) of adsorptive sintered compact (20) shown in Fig. 1 contain free adsorbent materials (free adsorbent agents) (1a) free-movably contained in voids (3) between resin structures (2). The free-movably means that powder adsorbent material is not fixed to resin structure (2) or a plurality of powder adsorbent materials are not fixed to each other, and that all kinds of movement such as shifting, rocking, vibration, rotation, extension/shrinkage, expansion/contraction, floating/sinking, etc., are possible.

Powder adsorbent materials (1a, 1b) of adsorptive sintered compact (20) shown in Fig. 1 are further provided with fixed adsorbent materials (1b) carried on resin structure (2). Fixed adsorbent materials (1b) are firmly fixed to surface (2a) of resin structure (2) or at least partly embedded inside resin structure (2). Namely, fixed adsorbent material (1b) can include: a first fixed adsorbent material (1b1) fixed to surface (2a) of resin structure (2); a second fixed adsorbent material (1b2) in which a part thereof (a part of surface) is embedded inside resin structure (2) and the remainder (the remainder of surface) is protruded from resin structure (2); and/or a third fixed adsorbent material (1b3) in which entire (entire surface) is embedded inside resin structure (2). In fixed adsorbent materials (1b), first and second fixed adsorbent materials (1b1, 1b2) in which surfaces thereof are exposed to voids (3) contribute to improvement in adsorption ability, and do not flow outside by fixing to resin structure (2) even they are small diameter. Although third fixed adsorbent material (1b3) is included in resin structure (2) and has low adsorption ability, the powder adsorbent materials (1a, 1b) having smaller diameter tend to be easily incorporated into resin structure (2), and fixed adsorbent material (1b3) is constituted by the powder adsorbent material having relatively small diameter, whereby it can prevent from clogging caused by large amount of powder adsorbent materials (1a, 1b) having smaller diameter remaining in voids (3) and can suppress increase in pressure loss.

In production steps of adsorptive sintered compact (20), the smaller particle diameter of powder adsorbent materials (1a, 1b), the more fixed to surface (2a) of resin structure (2) or at least a part thereof tends to be embedded inside resin structure (2), so that fixed adsorbent material (1b) tends to have relatively smaller particle diameter as compared with that of free adsorbent material (1a). On the contrary, free adsorbent material (1a) tends to have relatively large particle diameter. Therefore, when a part or all of free adsorbent material is recovered by cutting adsorptive sintered compact to measure mean diameter thereof, the mean diameter of free adsorbent material is generally larger than mean diameter of powder adsorbent material, as compared with that of powder adsorbent material used as raw material of adsorptive sintered compact (20). Mean diameter of free adsorbent material (1a) is preferably 5 µ or more and less than 150 µm, and further preferably 15 µ or more and 100 µm or less. Mean diameter of fixed adsorbent material (1b) is estimated to be 1 to 50 µm or so.

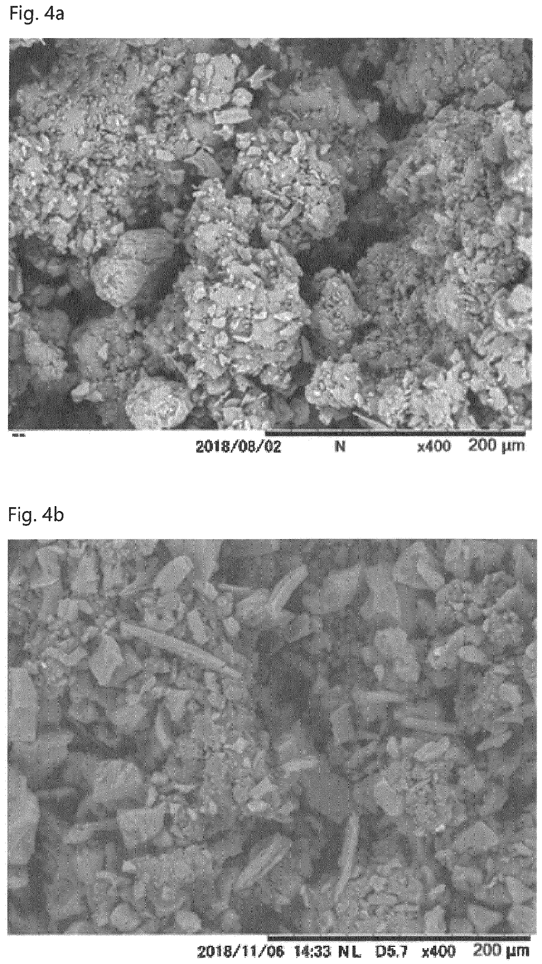

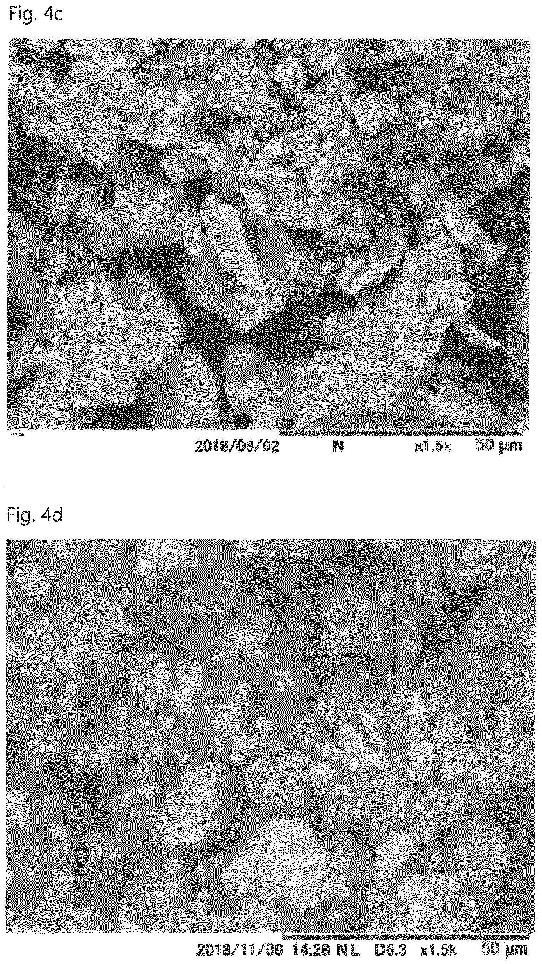

Fig. 4a to Fig. 4c show cross-sectional enlarged surface images of adsorptive sintered compacts (20) of the present invention made of, as powder adsorbent raw materials: powdered activated carbon (Fig. 2a) obtained by activating carbide of coconut shell with water vapor; powdered activated carbon (Fig. 2b) obtained by activating sawdust with a chemical (phosphoric acid); and powdered activated carbon (Fig. 2c) obtained by activating sawdust with a chemical (zinc chloride); which respectively photographed by an electron microscope of each magnification. In each case, it can confirm adsorptive sintered compact (20) comprising: resin structures (2) having smooth rounded surfaces (2a) with black-colored voids (3) formed into three-dimensional network shape; free adsorbent materials (1a) of powdered activated carbon provided inside resin structures (2); and fixed adsorbent materials (1b) of powdered activated carbon fixed to the resin structures (2). Black voids (3) inside resin skeleton constitute three-dimensional space in which free adsorbent materials (1a) can move freely. A part of fixed adsorbent materials (1b) is fixed to surface (2a) of resin structure (2), and a part thereof is fitted to a concave portion (2b) of surface (2a) and can be held in resin structure (2).

In the present invention, not only free adsorbent materials (1a) in voids (3) but also fixed adsorbent materials (1b) are provided in resin structures (2), so that higher adsorption performance can be maintained by increasing the adsorption surface and the saturated adsorption capacity. In other words, resin structure (2) alone has no adsorption ability otherwise a specific functional substance is added, so that the adsorption performance of entire adsorptive sintered compact (20) can be significantly improved by carrying fixed adsorbent materials (1b) on surfaces (2a) or inside of resin structures (2). Also, in the present invention, fixed adsorbent materials (1b) do not flow out from gap (4) between resin structures (2).

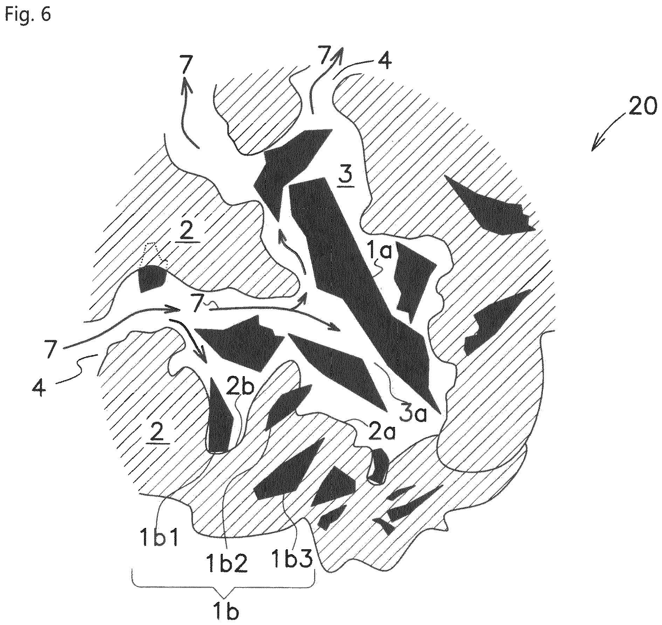

As can be confirmed from Fig. 4a to Fig. 4c, one or more free adsorbent material(s) (1a) is/are free-movably contained in voids (3). When two or more free adsorbent materials (1a) are contained in one void (3), these free adsorbent materials (1a) adjacent to each other form flow path (3a) (Fig. 1 and Fig. 6) for fluid (7) between free adsorbent materials (1a) of at least a part of voids (3) without fixing to each other to enable the full surface adsorption of free adsorbent materials (1a). Even if free adsorbent material (1a) once contacts with another free adsorbent material (1a) or resin structure (2), it can separate due to flow of fluid (7) and form flow path (3a) again.

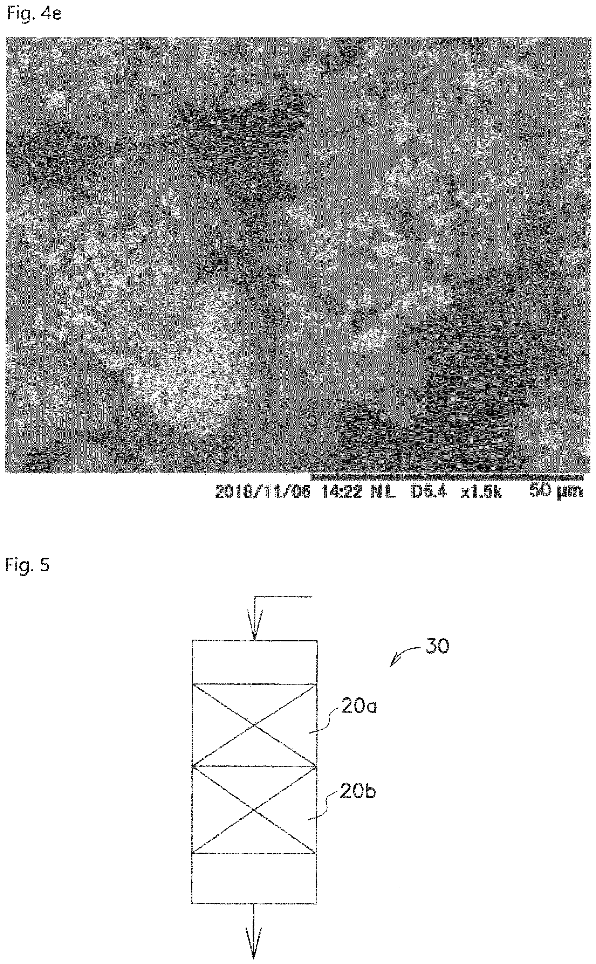

Fig. 4d and Fig. 4e show cross-sectional enlarged surface images (1,500-fold) photographed by an electron microscope that adsorptive sintered compacts (20) of the present invention are respectively made of, as raw material of powder adsorbent material, activated clay (Fig. 2d) and zeolite (Fig. 2e). From Fig. 4d and Fig. 4e, it can confirm adsorptive sintered compacts (20) comprising: grey resin structures (2) having smooth rounded surface (2a) in which black-colored voids (3) are formed in three-dimensional network shape; large-diameter powdered activated clay and zeolite as free adsorbent material (1a) provided inside resin structures (2); and small-diameter powdered activated clay and zeolite as fixed adsorbent material (1b) fixed to resin structures (2).

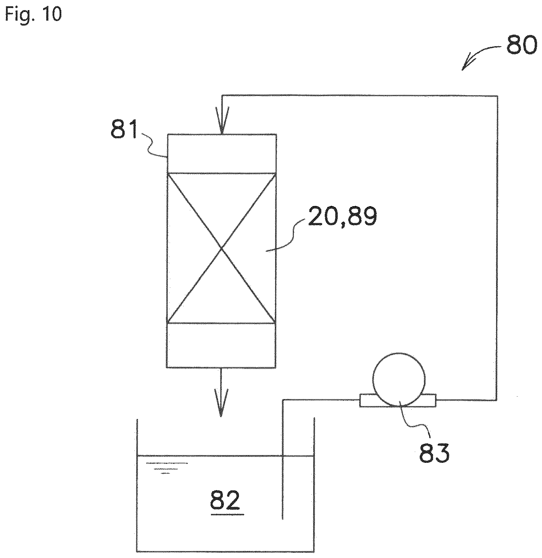

The adsorption apparatus of the present invention in which adsorptive sintered compact (20) is filled is formed by stacking a single layer or a plurality of layers of adsorptive sintered compact (20), for example, into a vessel as shown by reference numeral (81) in Fig. 10. As compared with the conventional technology in which granular adsorbent materials are directly filled in vessel, the adsorption apparatus of the present invention can maintain a certain form and voids (3) due to resin structures (2) having high mechanical strength without causing problems, such as pulverization and breakage of adsorbent materials in vessel and increase in pressure loss, at the time of transportation and use. Fig. 5 shows a two-layer (20a, 20b) adsorption apparatus (30). Even if each layer (20a, 20b) of adsorptive sintered compact (20) having such as columnar or prismatic or hollow shape is sequentially loaded or stacked in series, each layer (20a, 20b) does not mix with each other by maintaining rigidity. Although Fig. 5 shows two-layer (20a, 20b) adsorption apparatus (30), it is also possible to form an adsorption apparatus stacked into three or more layers.

The following explains an embodiment of the method for producing adsorptive sintered compact (20) according to the present invention using powdered activated carbon as raw material.

First, powdered activated carbon as raw material (powder adsorbent raw material) for constituting powder adsorbent materials (1a, 1b) mixes with thermoplastic resin as raw material of resin for constituting resin structure (2) to form adsorbent material mixture. Adsorbent material mixture may include other optional components if necessary. Content of powdered activated carbon is preferably 25 to 65 weight% in adsorbent material mixture, and more preferably 30 to 60 weight%. Content of thermoplastic resin is preferably 35 to 75 weight% in adsorbent material mixture, and more preferably 40 to 70 weight%. Mean diameter of powdered activated carbon is preferably less than 150 µm, more preferably 1 µm or more and less than 150 µm, further preferably 5 µm or more and less than 150 µm, and particularly preferably 15 µm or more and 100 µm or less. Particle diameter of thermoplastic resin is preferably 10 to 200 µm, and more preferably 30 to 80 µm. Powdered activated carbon hardly crushes, expands, or shrinks, and can form powder adsorbent materials (1a, 1b) while maintaining its original size. It can be also seen from for example Figs. 2a and 4a, and Figs. 2b and 4b showing images of the same magnification (400-fold) that the powder diameter of powder adsorbent raw material substantially is equal to that of powder adsorbent materials (1a, 1b) after production. Thermoplastic resin is preferably at least one kind selected from polypropylene, polyethylene, polyvinylidene fluoride and an ethylene-vinyl acetate copolymer. In the present invention, it is not necessary to use foaming agent. Water content of powdered activated carbon is preferably 30 weight% or less, more preferably 15 weight% or less, more preferably 8 weight% or less, and particularly preferably substantially free of water. If water content of powdered activated carbon is low, in the heating step mentioned later, the adsorptive sintered compact can be stably formed for a shorter time while suppressing energy consumption without lowering the temperature in vicinity of powdered activated carbon. Incidentally, as to using at least one kind of thermoplastic resin selected from polypropylene (PP), polyethylene (PE), polyvinylidene fluoride (PVDF) and an ethylene-vinyl acetate (EVA) copolymer preferably used in the present invention, these generally have poor water absorbency so that it is not necessary to consider the influence of water content in the method for producing adsorptive sintered compact (20) of the present invention.

Water content of powder adsorbent material can be measured as follows. 1g to 3g (w1) of powder adsorbent material is weighed and dried it at 110°C for a sufficient time until the mass change rate becomes 0.05%/min or less, and then the mass (w2) after drying is measured for the water content (%) obtained from 100 × (w1 - w2)/w2.

Next, adsorbent material mixture is introduced into a heating furnace, and adsorbent material mixture is heated at a temperature higher than the softening point of thermoplastic resin and lower than the melting point of powdered activated carbon, for example, at 90 to 180°C. By heating, the contact portions of plurality of thermoplastic resins are melted to form joint portions, and thermoplastic resins are fused to each other to form a skeleton for surrounding voids (3). In this case, most of powdered activated carbon does not bond to thermoplastic resin. Thereafter, it is cooled and solidified to form a three-dimensional network-shaped resin structure (2) in which free adsorbent materials (1a) are free-movably contained in voids (3). Resin structure (2) may have a cubic lattice-shaped three-dimensional network structure. This structure constitutes a high-strength adsorptive sintered compact (20) containing free adsorbent materials (1a) which consist of powdered activated carbon. Figs. 4a to 4c respectively illustrate embodiments of adsorptive sintered compacts (20) produced by the method of the present invention using powdered activated carbon as raw material. In the production of adsorptive sintered compact (20) of the present invention, by using powdered activated carbon and thermoplastic resin as resin raw materials preferably having lower water contents, it is possible to suppress the excessive void expansion due to vigorous evaporation and foaming of water, and voids (3) having appropriate sizes can be formed uniformly. By using raw material having low water content, in the heating step, heat is uniformly transferred from the outside near a heating source to the inside distal without being affected by moisture, so that there is no temperature difference and entire thermoplastic resins can be uniformly melted. In addition, almost no water is evaporated and released from powdered activated carbon, and no external force is applied to thermoplastic resin at the stage of forming resin structures (2) so that there is no deforming, whereby voids (3) can be formed and maintained to substantially uniform size within a predetermined range without expansion. Further, from the viewpoint of forming such an appropriate dimension and voids (3) uniformly, the particle diameter of thermoplastic resin is preferably 10 to 200 µm, and more preferably 30 to 80 µm.

In the above-mentioned embodiment of the production method, powdered activated carbon is used as raw material, but even if powdered activated clay or zeolite is used as raw material, adsorptive sintered compact (20) can be produced by the same production method as mentioned above (Fig. 4d and Fig. 4e).

An embodiment in which fluid (7) as liquid or gas is passed through adsorptive sintered compact (20) of the present invention will be explained below by referring to Fig. 6.

When fluid (7) containing substance(s) to be treated is passed through an adsorption apparatus (not shown in the drawing) stacked a single-layer adsorptive sintered compact (20), fluid (7) is introduced into voids (3) passing through gap (4) between resin structures (2). At this time, free adsorbent material (1a) is in a non-bonding state to resin structure (2), and plurality of free adsorbent materials (1a) are not fixed or bonded to each other, so that, as shown in Fig. 6, free adsorbent materials (1a) are floated or swung in voids (3) by flowing fluid (7), and entire surface of free adsorbent material (1a) comes into direct contact with fluid (7) whereby it can certainly capture and adsorb the substance. Further, by flow path (3a) formed between free adsorbent materials (1a), the adsorption area is increased, and flow of fluid (7) can be ensured whereby increase in pressure loss can be prevented in adsorptive sintered compact (20).

Also, substance to be treated in fluid (7) is captured and adsorbed by contacting fluid (7) with surface (2a) of resin structure (2) or fixed adsorbent material (1b) fixed inside thereof. Fluid (7) treated in voids (3) passes through gap (4) and is continuously introduced into other voids (3). The same adsorption treatment is repeated in the plurality of other voids (3), and finally fluid (7) is flown out to the outside of resin structure (2), that is, to the outside of adsorption apparatus. Since gap (4) is small, freely moving free adsorbent material (1a) is retained in voids (3) between resin structures (2) and does not flow out to the outside of adsorptive sintered compact (20) .

Adsorptive sintered compact (20) of the present invention can contain a high content of powder adsorbent material excellent in adsorption performance, and therefore, it can make adsorption treatment of substance(s) with high efficiency. When filling in vessel such as cartridge for the use of filter, the adsorption apparatus such as filter can be miniaturized due to the small size vessel, and the transportability and storability can be made extremely good because adsorptive sintered compact (20) of the present invention is also excellent in strength.

In adsorptive sintered compact (20) of the present invention, lowering in adsorption performance due to increase in pressure loss can be suppressed, and adsorption performance can be maintained for a long period of time while retaining a predetermined treatment amount, so that reduction of running costs can be accomplished.

In adsorptive sintered compact (20) of the present invention, powdered activated carbon excellent in adsorption performance can be integrally molded with a desired shape and size as a sintered compact, and a continuous adsorption treatment with various forms becomes possible. Further, multiple functional adsorption apparatus can be provided because it is also possible to load the plurality of layers having different adsorption characteristics in vessel.

In addition, adsorptive sintered compact (20) of the present invention can be used for a batch type adsorption treatment. In batch type adsorption treatment using powder adsorbent material, the powder adsorbent material is easily scattered in air so that handling property thereof is not good and there is a case that a local exhaust equipment is required for preventing the deterioration of working environment due to powder dust, however, the adsorptive sintered compact (20) of the present invention also has an advantage in handling property.

### EXAMPLES

Examples of adsorptive sintered compact (20) according to the present invention will be explained as follows.

### [1] Pressure loss test

With regard to pressure loss of adsorptive sintered compact (20) (Examples 1 to 4) of the present invention, the following experiment was carried out with powder objects (69) (Comparisons 1 to 4 and Comparisons 1' and 2').

### [1-1] Production of adsorptive sintered compact (20) and powder object (69)

As raw materials of powder adsorbent materials (1a, 1b), 0.109 weight part of powdered activated carbon (AC) (Fig. 2a) having about 30 µm mean diameter obtained by activating coconut shell carbide with water vapor and 0.164 weight part of Polyethylene (PE) powder as resin raw material of resin structure (2) were mixed, and heated and sintered at about 125°C to obtain adsorptive sintered compact (20) (Fig. 4a) (Example 1) of the present invention containing 40 weight% of powder adsorbent materials (1a, 1b). Similarly, as raw materials of powder adsorbent materials (1a, 1b), 0.109 weight part of powdered AC (Fig. 2b) having about 42 µm mean diameter obtained by activating sawdust with chemical (phosphoric acid) and 0.109 weight part of PE powder as resin raw material of resin structure (2) were mixed, and heated and sintered at about 125°C to obtain adsorptive sintered compact (20) (Fig. 4b) (Example 2) of the present invention containing 40 weight% of powder adsorbent materials (1a, 1b). Water contents of powdered ACs as raw material used in Examples 1 and 2 were each about 7 weight%. As raw materials of powder adsorbent materials (1a, 1b), 0.139 weight part of powdered activated clay (Fig. 2d) having about 11 µm mean diameter and 0.139 weight part of PE powder as resin raw material of resin structure (2) were mixed, and heated and sintered at about 125°C to obtain adsorptive sintered compact (20) (Fig. 4d) (Example 3) of the present invention containing 50 weight% of powder adsorbent materials (1a, 1b). As raw materials of powder adsorbent materials (1a, 1b), 0.139 weight part of zeolite (Fig. 2e) having about 35 µm mean diameter and 0.139 weight part of PE powder as resin raw material of resin structure (2) were mixed, and heated and sintered at about 125°C to obtain adsorptive sintered compact (20) (Fig. 4d) (Example 4) of the present invention containing 50 weight% of powder adsorbent materials (1a, 1b). Water contents of powdered activated clay and zeolite as raw materials used in Examples 3 and 4 were each about 7 weight%.

0.109 weight part of powdered AC (Fig. 2a) having about 30 µm mean diameter obtained by activating coconut shell carbide with water vapor was produced as powder object (69) (Comparison 1). 32 µm or less of powdered AC was classified and removed by vibration classifier (manufactured by Fritsch Co., Ltd.) from powdered AC (Fig. 2a) having about 30 µm mean diameter obtained by activating coconut shell carbide with water vapor to obtain 0.109 weight part of powdered AC as powder object (69) (Comparison 1'). 0.109 weight part of powdered AC (Fig. 2b) having about 42 µm mean diameter obtained by activating sawdust with chemical (phosphoric acid) was produced as powder object (69) (Comparison 2). 32 µm or less of powdered AC was classified and removed by vibration classifier from powdered AC (Fig. 2b) having about 42 µm mean diameter obtained by activating sawdust with chemical (phosphoric acid) to obtain 0.109 weight part of powdered AC as powder object (69) (Comparison 2'). 0.109 weight part of powdered activated clay (Fig. 2d) having about 11 µm mean diameter was produced as powder object (69) (Comparison 3). 0.109 weight part of zeolite (Fig. 2e) having about 35 µm mean diameter was produced as powder object (69) (Comparison 4).

### [1-2] Test method

### <Examples 1 to 4>

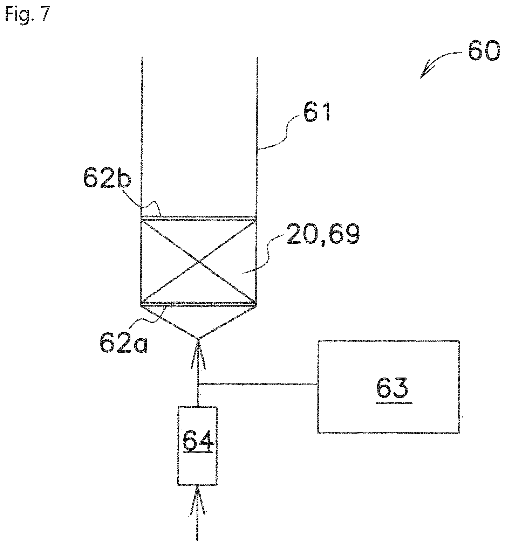

A frit (filter for preventing from powder leakage) (62a) was arranged in each syringe (61) having volume of about 3 ml, each adsorptive sintered compact (20) of Examples 1 to 4 was loaded thereon, and further frit (62b) was arranged to make a pressure loss test apparatus (60) in Fig. 7. The pressure difference of adsorptive sintered compact (20) when air adjusted to 0.2 L/min by a flow meter (64) was passed through adsorptive sintered compact (20), was measured by a pressure gauge (AP-53A manufactured by KEYENCE CORPORATION) (63) with regard to each of Examples 1 to 4.

### <Comparisons 1 to 4 and Comparisons 1' and 2'>

In the same test method as the above Examples except that syringes (61) were filled with each of powder object (69) of Comparisons 1 to 4 and Comparisons 1' and 2' in place of adsorptive sintered compact (20), the differential pressure of each Comparison was measured.

### [1-3] Test results and consideration

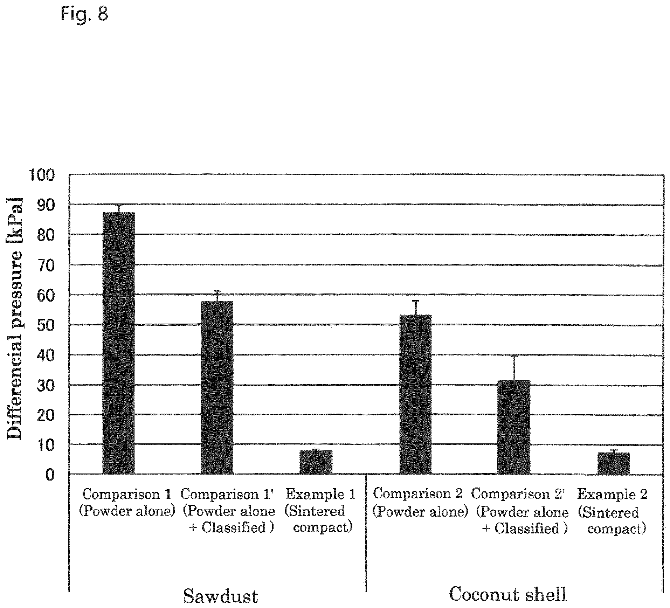

As shown in pressure loss test results of Fig. 8 in which the name of test object is shown on the horizontal axis and the differential pressure [kPa] is shown on the vertical axis, Comparisons 1 and 2 in filled with powdered AC showed high differential pressure value of 50 kPa or more, and Comparisons 1' and 2' in which powdered AC of 32 µm or less was classified and removed also showed high differential pressure value of 30 kPa or more. Comparisons 1 and 2 were filled with powdered AC containing fine powder of 32 µm or less with high density, and Comparisons 1' and 2' were also filled with powdered AC with high density, so that space between powders is small whereby these were high pressure loss. To the contrary, adsorptive sintered compacts (20) according to the present invention of Examples 1 and 2 each showed low differential pressure value of 10 kPa or less in spite of containing resin structure (2) in addition to the same amount of powdered AC as Comparisons. Comparisons 3 and 4 in which powder object (69) of powdered activated clay and zeolite was filled showed high differential pressure value of 29 kPa and 97 kPa, respectively. Comparisons 3 and 4 were high pressure loss since fine powder was filled with high density and space between powders was small. To the contrary, adsorptive sintered compacts (20) according to the present invention of Examples 3 and 4 each showed low differential pressure value of 10 kPa or less in spite of containing resin structure (2) in addition to the same amount of powdered activated clay and zeolite as Comparisons 3 and 4. Accordingly, in Examples 1 to 4 of the present invention, it was confirmed that the lower pressure loss treatment was realized. Further, adsorptive sintered compacts (20) of Examples 1 to 4 were also excellent in strength.

### [2] Liquid flow adsorption test

With regard to the liquid flow adsorption performance of adsorptive sintered compacts (20) (Examples 5 and 6) of the present invention, the following test was carried out together with granular objects (89) (Comparisons 5 and 6) using granular activated carbon.

### [2-1] Production of adsorptive sintered compact (20) and granular object (89)

As raw materials of powder adsorbent materials (1a, 1b), 0.076 weight part of powdered AC (Fig. 2a) having about 30 µm mean diameter obtained by activating coconut shell carbide and 0.177 weight part of PE powder as resin raw material of resin structure (2) were mixed, and heated and sintered at about 125°C to obtain high strength adsorptive sintered compact (20) (Fig. 4a) (Examples 5) of the present invention containing 30 weight% of powder adsorbent materials (1a, 1b). Similarly, as raw materials for constituting powder adsorbent materials (1a, 1b), 0.076 weight part of powdered AC (Fig. 2b) having about 42 µm mean diameter obtained by activating sawdust with chemical (phosphoric acid) and 0.177 weight part of PE powder as resin raw material of resin structure (2) were mixed, and heated and sintered at about 125°C to obtain high strength adsorptive sintered compact (20) (Fig. 4b) (Examples 6) of the present invention containing 30 weight% of powder adsorbent material (1a, 1b). Water content of powdered AC used in Examples 5 and 6 was about 8 weight%.

0.076 weight part of granular activated carbon having about 800 µm mean diameter obtained by activating coconut shell carbide with water vapor and 0.177 weight part of PE beads having 100 µm diameter were mixed to obtain granular object (89) (Comparison 5). In large diameter granular activated carbon having about 800 µm diameter, adsorptive sintered compact of the present invention in which AC was free-movably arranged in voids between resin structures could not be formed, and number of grains was extremely small as compared with that of powder so that voids without AC were formed with a large number, so that the same amount of PE beads as that of PE powder was used in order to meet the same conditions as in Example 5 as much as possible. Similarly, 0.076 weight part of granular activated carbon having about 800 µm mean diameter obtained by activating sawdust raw material with chemical (phosphoric acid) and 0.177 weight part of PE beads having 100 µm diameter were mixed to obtain granular object (89) (Comparison 6). Incidentally, 800 µm mean diameter corresponds to particle diameter of granular activated carbon frequently used in a liquid phase treatment.

### [2-2] Test method

### <Examples 5 and 6>

According to liquid flow adsorption test apparatus (80), 20 ml of methylene blue solution having concentration of 1,200 mg/l stored in container (82) was fed by a tube pump (83) with 3.2 ml/min to vessel (81) having inner diameter of 8.6 mm loaded with each of adsorptive sintered compacts (20) of Examples 5 and 6 and was circulated (Fig. 10). After collecting every 0.1 ml of the circulating fluid at constant intervals and diluting them 100 times, the absorbance at wavelength of 665 nm was measured by a spectrophotometer (UV-1700 manufactured by Shimadzu Corporation), and residual concentration of methylene blue in the circulating fluid was obtained for Examples 5 and 6.

### <Comparisons 5 and 6>

In the same test method as the above Examples 5 and 6 except that granular objects (89) of Comparisons 5 and 6 filled therewith in each vessel (81) were used in place of adsorptive sintered compact (20), residual concentration of methylene blue was obtained for Comparisons 5 and 6.

### [2-3] Test results and consideration

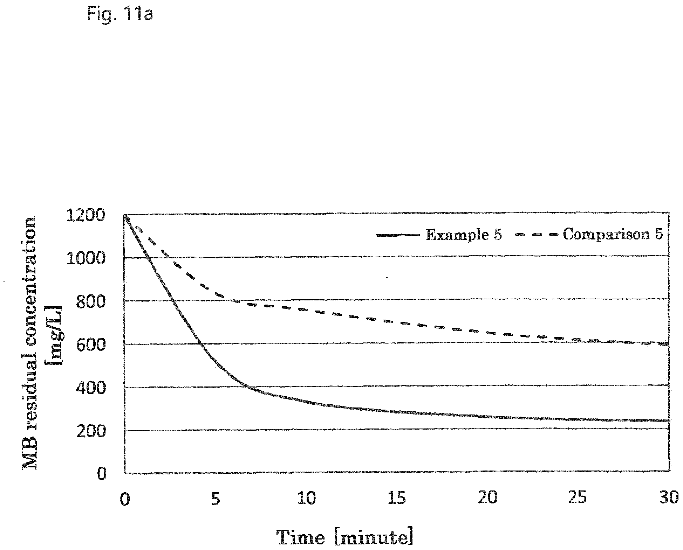

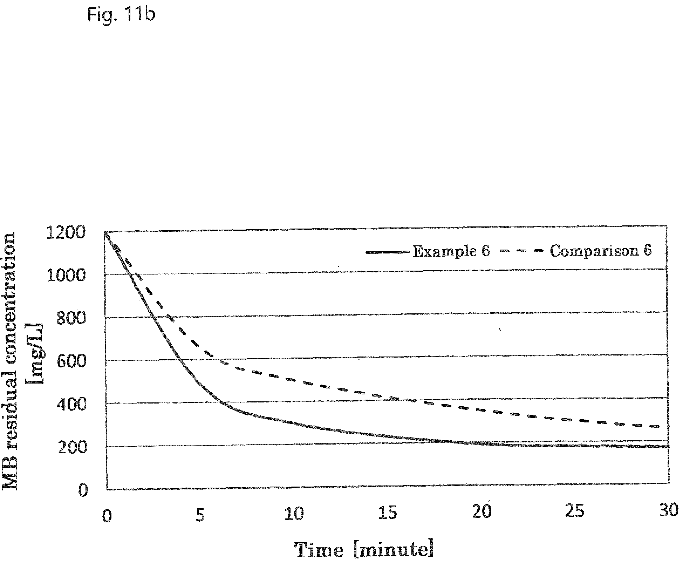

Fig. 11a shows test results of Example 5 and Comparison 5, and Fig. 11b shows test results of Example 6 and Comparison 6, and time [min] is shown on the horizontal axis, and residual concentration [mg/l] of methylene blue is shown on the vertical axis, respectively. For example, when residual concentrations of methylene blue after 10 min were compared to each other, from Fig. 11a, Comparison 5 was about 780 mg/l, and Example 5 decreased to about 300 mg/l. Also, from Fig. 11b, Comparison 6 was about 500 mg/l, and Example 6 decreased to 300 mg/l. Therefore, as compared with Comparisons 5 and 6 containing granular activated carbon alone having 800 µm diameter, Examples 5 and 6 of the present invention containing 30 weight% of powder adsorbent materials (1a, 1b) could be confirmed that the adsorption performance of liquid, particularly, decoloration property was excellent. Also, adsorptive sintered compacts (20) of Examples 5 and 6 were excellent also in strength.

### [3] Gas flow adsorption test

With regard to gas flow adsorption performance of adsorptive sintered compact (20) (Example 5) of the present invention, the following test was carried out together with granular objects (99) (Comparisons 7 and 8) using granular activated carbon.

### [3-1] Production of adsorptive sintered compact (20) and granular object (99)

Adsorptive sintered compact (20) of Example 5 was obtained by the same method as mentioned above. 0.076 weight part of granular activated carbon having about 2,000 µm mean diameter obtained by activating coconut shell carbide with water vapor and 0.177 weight part of PE beads having 100 µm diameter were mixed to obtain granular object (99) (Comparison 7). In large diameter granular activated carbon having about 2,000 µm particle diameter, adsorptive sintered compact of the present invention in which AC was free-movably arranged in voids between resin structures cannot be formed, and number of grains was extremely small as compared with that of powder so that voids without activated carbon were formed with large number, so that the same amount of PE beads as that of PE powder was used in order to meet the same conditions as in Example 5 as much as possible. Granular object (99) (Comparison 8) containing only 0.076 weight part of granular activated carbon having about 2,000 µm mean diameter was obtained by activating coconut shell carbide with water vapor. Incidentally, 2,000 µm mean diameter corresponds to particle diameter of granular activated carbon frequently used in gas phase treatment.

### [3-2] Test method

### <Example 5>

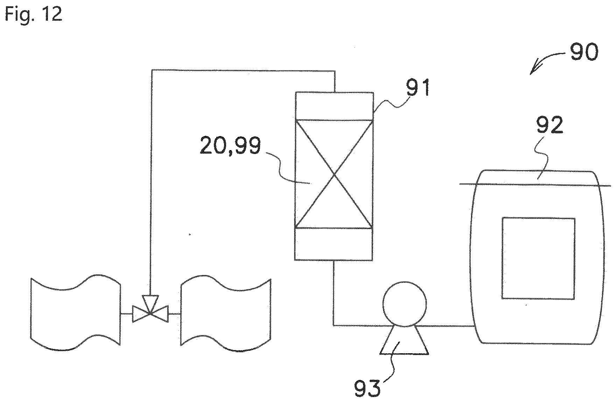

According to a gas flow adsorption test apparatus (90) shown in Fig. 12, cyclohexane gas was supplied at 0.2 ml/min by a diaphragm pump (93), from a gas capturing bag (92) adjusted to about 100 ppm of cyclohexane gas by arranging therein a cyclohexane impregnated source, to a vessel (91) having inner diameter of 8.6 mm loaded with adsorptive sintered compact (20) (Example 5). Outlet gas after passing through adsorptive sintered compact (20) was collected in amount of 2L in 10 minutes, and cyclohexane concentration of every 10 minutes was measured by a gas detector tube (GASTEC CORPORATION 102L). In addition, odor at outlet gas was evaluated by a sensory test.

### <Comparisons 7 and 8>

In the same test method as the above Example 5 except that granular objects (99) of Comparisons 7 and 8 filled therewith in each vessel (81) were used in place of adsorptive sintered compact (20), the cyclohexane concentration was measured, and the sensory test was carried out.

### [3-3] Test results and consideration

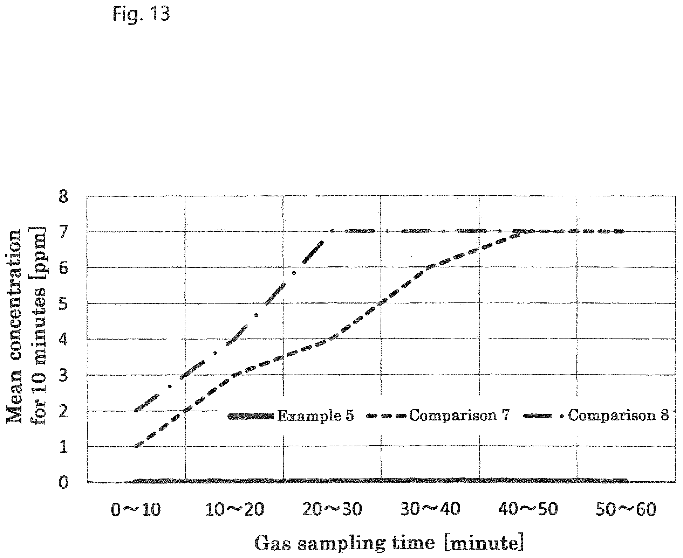

Fig. 13 shows test results of Example 5 and Comparisons 7 and 8, and time [min] is shown on the horizontal axis, and cyclohexane concentration [ppm] is shown on the vertical axis. In Comparisons 7 and 8, cyclohexane broke through from first 10 minutes, whereas in Example 5, cyclohexane did not break through even after 60 minutes, and the adsorption was completely maintained. In addition, as a result of sensory test, in Comparisons 7 and 8, cyclohexane odor of outlet gas was felt from the first 10 minutes, whereas in Example 5, no odor was felt. Therefore, Example 5 of the present invention containing 30 weight% of powder adsorbent materials (1a, 1b) could be confirmed that it was excellent in adsorption performance of gas, particularly, deodorizing property and toxic gas removing characteristics.

### [4] Sinterability and strength test

Sinterability and strength of adsorptive sintered compact (20) containing powder adsorbent materials (1a, 1b) were confirmed.

### [4-1] Manufacture and test method of adsorptive sintered compact (20)

Powdered AC (Fig. 2b) having about 42 µm mean diameter obtained by activating sawdust with chemical (phosphoric acid) as raw material for constituting powder adsorbent materials (1a, 1b) and PE powder for forming resin structure (2) were mixed so that adsorptive sintered compact (20) became 0.273 g, and heated and sintered at about 125°C, whereby adsorptive sintered compacts (20) (Examples 7 and 8) containing 50 and 60 weight% of powder adsorbent materials (1a, 1b) were obtained, respectively. The reason why powdered AC of sawdust is used is that, in the above Examples 1 to 6, sawdust has relatively poor strength as compared with the other raw materials, and if high sinterability and high strength can be confirmed in sawdust powdered AC, it is naturally predictable that sufficient sinterability and strength can be also obtained from the other coconut shell powdered AC, powdered activated clay, and zeolite.

### [4-2] Test method

With regard to Examples 7 to 8, sinterability was confirmed by visual inspection of appearance and strength by touching hands, respectively.

### [4-3] Test results and consideration

Adsorptive sintered compacts (20) containing 30 weight% (Example 6), 40 weight% (Example 2), 50 weight% (Example 7) and 60 weight% (Example 8) of powder adsorbent materials (1a, 1b) each could be taken out from a heating furnace in a state maintaining its shape, and the shape did not change even when pressed strongly, so that sufficient strength could be confirmed.

### [5] Batch type liquid phase adsorption test

With regard to batch type liquid phase adsorption performance of adsorptive sintered compact (20) of the present invention, the following test was carried out together with powdered activated clay (Fig. 2d).

### [5-1] Manufacture of adsorptive sintered compact (20)

As raw materials of powder adsorbent materials (1a, 1b), 0.197 weight part of powdered activated clay (Fig. 2d) having about 11 µm mean diameter and 0.131 weight part of PE powder as resin raw material of resin structure (2) were mixed, and heated and sintered at about 125°C to obtain adsorptive sintered compact (20) of the present invention (Examples 9) containing 60 weight% of powder adsorbent materials (1a, 1b). Water content in the powdered activated clay was about 7 weight%.

### [5-2] Test method

### <Example 9>

In 100 ml Erlenmeyer flask was charged adsorptive sintered compact (20) of Example 9, and 50 ml of caffeine aqueous solution having concentration of 100 mg/l was added thereto, and flask was put with a rubber stopper and set on a shaker and shook at normal temperature (20°C) with 200 rpm until adsorption reaches almost equilibrium. The liquid after shaking was collected, caffeine was separated using a high performance liquid chromatograph device (Chromaster (Registered Trademark) manufactured by Hitachi High-Tech Science Corporation), and residual concentration of caffeine for Example 9 was measured from absorbance at wavelength of 280 nm to obtain removal rate of caffeine by adsorption.

### <Comparison 9>

In the same test method as the above Example 9 except that 0.197 weight part of powdered activated clay (Fig. 2d) was used in place of adsorptive sintered compact (20), the removal rate of caffeine by adsorption was obtained.

### [4-3] Test results and consideration

Both of Example 9 and Comparison 9 showed equal value of 97% in the removal rate of caffeine by adsorption. Therefore, it could be confirmed that adsorption performance of powder adsorbent materials (1a, 1b) was highly maintained even in adsorptive sintered compact (20). In addition, adsorptive sintered compact (20) of Example 9 was also excellent in strength.

### [6] Batch type gas phase adsorption test

With regard to batch type gas phase adsorption performance of adsorptive sintered compact (20) of the present invention, the following test was carried out together with chemical-supported zeolite.

### [6-1] Manufacture of adsorptive sintered compact (20)

As raw materials of powder adsorbent materials (1a, 1b), 0.055 weight part of chemical-supported zeolite pulverized to about 35 µm mean diameter and 0.055 weight part of PE powder as resin raw material of resin structure (2) were mixed, and heated and sintered at about 125°C to obtain high strength adsorptive sintered compact (20) (Example 10) of the present invention containing 50 weight% of powder adsorbent materials (1a, 1b). Water content of chemical-supported zeolite was about 7 weight%.

### [6-2] Test method

### <Example 10>

In 3L odor bag (manufactured by Omi Odor Air Service Co., Ltd.) made of polyester were placed adsorptive sintered compact (20) of Example 10 and a filter paper piece of about 3 cm square, and 3L of clean air was added therein and bag was sealed with rubber stopper. 3% dimethyl sulfide (hereinafter referred to as DMS) solution was injected into odor bag with micro syringe so that internal DMS concentration became 200 mg/m³ to impregnate it into filter paper piece, and injection port was sealed with cellophane tape. DMS was vaporized inside odor bag, and bag was left at rest at normal temperature (20°C) until adsorption was almost equilibrium. Rubber stopper was opened and DMS concentration in odor bag was measured using gas detecting tube No. 77 manufactured by GASTEC CORPORATION to obtain removal rate of DMS by adsorption.

### <Comparison 10>

In the same test method as the above Example 10 except that 0.055 weight part of chemical-supported zeolite pulverized to about 35 µm mean diameter was used in place of adsorptive sintered compact (20), the removal rate of DMS by adsorption was obtained.

### [6-3] Test results and consideration

Removal rate of DMS by adsorption of Examples 10 was 96%, and removal rate of DMS by adsorption of Comparison 10 was 97%, which were almost the same values. Therefore, it could be confirmed that adsorption performance of powder adsorbent materials (1a, 1b) was highly maintained even in adsorptive sintered compact (20). In addition, adsorptive sintered compact (20) of Example 10 was also excellent in strength.

### [7] Conclusion of Examples

From the above Examples, it could be confirmed that adsorptive sintered compact (20) of the present invention had high strength and exhibited excellent adsorption performance such as decoloration and deodorization, even at low pressure loss in liquid and gas.

### UTILIZABILITY IN INDUSTRY

The adsorptive sintered compact, a production method therefor and the adsorption apparatus of the present invention can be used for various uses such as air purification, dioxin removal, flue gas desulfurization and denitrification, waste gas and liquid treatment of factory, advanced water purification, purification of chemicals, decolorization of foods and beverages, water purifiers for home use, air purifiers, refrigerator deodorants, gas masks, etc.

### EXPLANATION OF REFERENCE NUMERALS

(1a, 1b) ··Powder adsorbent material, (1a) ··Free adsorbent material, (1b) ··Fixed adsorbent material, (2) ··Resin structure, (2a) ··Surface, (2b) ··Concave portion, (3) ··Voids, (20) ··Adsorptive sintered compact, (30) ··Adsorption apparatus,

## Claims

1. An adsorptive sintered compact which comprises:
powder adsorbent materials; and
a resin structure in which voids are formed in a three-dimensional network;
wherein the powder adsorbent materials include:
a free adsorbent material free-movably contained in the voids between the resin structures; and
a fixed adsorbent material fixed to a surface of the resin structure and/or at least partly embedded inside the resin structure; and
the powder adsorbent materials are at least one selected from powdered activated carbon, powdered activated clay and zeolite.

2. The adsorptive sintered compact according to Claim 1, wherein a mean diameter of the powder adsorbent materials is less than 150 µm.

3. The adsorptive sintered compact according to Claim 1 or 2, wherein the powder adsorbent materials are contained in an amount of 25 to 65 weight%.

4. The adsorptive sintered compact according to any one of Claims 1 to 3, wherein a resin raw material of the resin structure is at least one thermoplastic resin selected from polypropylene (PP), polyethylene (PE), polyvinylidene fluoride (PVDF) and ethylene-vinyl acetate (EVA) copolymer.

5. The adsorptive sintered compact according to any one of Claims 1 to 4, wherein a particle diameter of a thermoplastic resin is 10 to 200 µm.

6. The adsorptive sintered compact according to any one of Claims 1 to 5, wherein a plurality of free adsorbent materials are free-movably contained in at least a part of the voids, and
the plurality of the adjacent free adsorbent materials form a flow path of a fluid between free adsorbent materials in at least a part of voids without a fixation of the adjacent free adsorbent materials to each other.

7. The adsorptive sintered compact according to any one of Claims 1 to 6, wherein the powder adsorbent material is the powdered activated carbon having a sharp portion.

8. The adsorptive sintered compact according to any one of Claims 1 to 7, which is used for adsorbing a substance to be treated in a fluid.

9. An adsorption apparatus which comprises a single or a plurality of layers of the adsorptive sintered compact according to any one of Claims 1 to 8 being loaded in a vessel.

10. A method for producing an adsorptive sintered compact which comprises steps of:
mixing a powder adsorbent material which is at least one selected from powdered activated carbon, powdered activated clay and zeolite, with a thermoplastic resin to form an adsorbent material mixture;
heating the adsorbent material mixture at a temperature higher than a softening point of the thermoplastic resin and lower than a melting point of a raw material of the powder adsorbent material; and
forming a resin structure in which a plurality of the thermoplastic resins are fused and solidified by cooling to form voids in a three-dimensional network and a free adsorbent material is free-movably contained in the void.

11. The method according to Claim 10, wherein a mean diameter of the powder adsorbent material is less than 150 µm.

12. The method according to Claim 10 or 11, wherein a content of the powder adsorbent material is 25 to 65 weight% in the adsorbent material mixture.

13. The method according to any one of Claims 10 to 12, wherein the thermoplastic resin is at least one selected from polypropylene (PP), polyethylene (PE), polyvinylidene fluoride (PVDF) and ethylene-vinyl acetate (EVA) copolymer.

14. The method according to any one of Claims 10 to 13, wherein a particle diameter of the thermoplastic resin is 10 to 200 µm.
